# EUROPEAN PATENT APPLICATION

(11) **EP 2 878 663 A1**
(43) Date of publication of application: **03.06.2015**
(21) Application number: 13846368.2
(22) Date of filing: 17.10.2013
(51) Int. Cl.: C12M 1/16, C12M 3/02

(54) **CELL CULTURE CONTAINER HAVING DUAL STRUCTURE, AND CIRCULATION CULTURE SYSTEM USING SAME**

(30) Priority: 19.10.2012 KR 20120116653
(71) Applicant: Korea Research Institute of Bioscience and Biotechnology, Daejeon 305-333 (KR)
(72) Inventor: JUNG, Cho-Rok, Daejeon 305-806 (KR); LIM, Jung Hwa, Daejeon 305-806 (KR); IM, Dong-Soo, Daejeon 305-806 (KR)
(74) Representative: Vossius & Partner Patentanwälte Rechtsanwälte mbB
(86) International application number: PCT/KR2013/009309
(87) International publication number: WO 2014/062022

(57) **Abstract**

The present invention relates to a cell culture container comprising: an outer container of which an upper surface is open and into which a culture solution can be put; a container cover coupled to the upper end of the outer container to prevent the penetration of microorganisms; and an inner container which is placed inside the outer container, can put a culture solution, and can be separated from the outer container. The present invention relates to a cultivation culture system comprising: a plurality of the cell culture containers; circulation pipes for connecting each of the adjacent cell culture containers so as to allow the plurality of the cell culture containers to be interconnected and connecting a pair of the cell culture containers placed at both ends; and a circulation portion for supplying a culture solution and gas to a cell culture portion so as to circulate the same.

## Description

### BACKGROUND

### Field of the Invention

The present invention relates to a cell culture container and a circulation culture system in which cells and tissues of human or animals/plants, and microorganisms can be cultured.

### Discussion of Related Art

In general, a culture container which is used when animal tissues, plant tissues, plants, or the like are cultured includes a container body of which the upper surface is open and into which a culture medium can be held and a cover coupled to the container body to prevent the culture medium from being contaminated by the penetration of microorganisms or the like, thereby allowing the tissues of animals/plants or plants or the like to be held therein and cultured after the container is closed with the cover.

In addition, the container body and the cover is made of a transparent material through which light is transmitted, and the culture medium which is injected into the culture container is separated into a solid culture medium and a culture medium, which is selected and used according to the characteristics of the tissues or the plants to be cultured. Under such environments, various types of technology for culture containers have been developed up to the present time.

In connection therewith, Korean Patent No. 10-0679248 discloses a culture container provided with a partition and sharing a culture medium and Korean Patent Application No. 10-1999-0041759 discloses a culture container for preventing the penetration of outer microorganisms. However, conventional culture containers described above are disposable and used for culturing only single cells, but cannot be used for culturing two different types of cells simultaneously and do not allow the continuous inflow/discharge of the culture medium or gas.

Further, since the cells cultured through conventional cell culture techniques are monolayer cells in which cells are spread out in a two-dimensional direction, the cells cannot be constructed into three-dimensional tissue identical to that in a living body and cannot maintain the specific function that the cells have a the living body for a long time. Thus, there is the problem in that accuracy of simulation cannot be guaranteed.

### SUMMARY OF THE INVENTION

### Technical Problem

An object of the present invention is to provide a dual structure cell culture container including an outer container which can hold a culture medium, and an inner container which is comprised of a different material with that of the outer container and placed inside the outer container.

An object of the present invention is to provide a circulation culture system comprised of a plurality of dual culture containers, which allow a three-dimensional culture and are connected to each other through circulation conduits, thereby making it possible to set a circulation conduit and flow rate as necessary and selectively control the circulation of a culture medium.

### Technical Solution

The present invention provides a cell culture container which includes an outer container of which the upper surface is open and into which a culture medium can be held; a container cover coupled to an upper end of the outer container to prevent the penetration of microorganisms; and an inner container which is placed inside the outer container, can hold a culture medium, and can be separated from the outer container.

The present invention provides a circulation culture system which includes a plurality of cell culture containers; circulation conduits for connecting each of the adjacent cell culture containers so as to allow the plurality of the cell culture containers to be interconnected and for connecting a pair of the cell culture containers placed at both ends; and a circulation section to supply a culture medium and gas to a cell culture portion so as to circulate the same.

### Advantageous Effects

According to the present invention, since the inner container which is comprised of a different material than that of the outer container is placed inside the outer container into which the culture medium can be held, it is possible to simultaneously culture adherent cells and non-adherent cells or two different types of non-adherent cells, and since the outer container and the inner container can be separated from each other, the inner container to which cells are adhered so that they are cultured is separated from the outer container, thereby allowing convenience in transferring the inner container to other culture environments.

Further, according to the present invention, three-dimensional culture is made possible thereby a culture condition similar to that in a living body can be provided, and the circulation of the culture medium can be sequentially, selectively and quantitatively controlled.

Further, in addition to providing cell culture, the present invention also makes available a device for controlling sequentially, selectively and quantitatively the transfer of various types of liquids in circulation facilities such as laboratories, factories manufacturing chemical products, waste water disposal facilities or the like.

### BRIEF DESCRIPTION OF THE DRAWINGS

FIG. 1 is an exploded perspective view of a cell culture container according to the present invention.
FIG. 2 is a perspective view of an outer container according to the present invention.
FIG. 3 is a front view of the outer container according to the present invention.
FIG. 4 is a right side view of the outer container according to the present invention.
FIG. 5 is a sectional view of the cell culture container according to the present invention.
FIG. 6 is a perspective view of an inner container according to the present invention.
FIG. 7 is a configuration view of a circulation culture system according to the present invention.
FIG. 8 is a configuration view of a circulation culture system according to an embodiment of the present invention.
FIG. 9 is a view showing the results after various cells have been cultured using the circulation culture system according to the embodiment of the present invention.

### DETAILED DESCRIPTION OF PREFERRED EMBODIMENTS

The present invention is configured to have a cell culture container including an outer container of which the upper surface is open and into which a culture medium can be held; a container cover coupled to an upper end of the outer container to prevent the penetration of microorganisms; and an inner container which is placed inside the outer container, can hold a culture medium, and can be separated from the outer container.

The outer container is provided at one side surface thereof with an inlet portion through which a culture medium or gas can flow into the outer container and at the other side surface thereof with an outlet portion through which the culture medium or gas which has flowed into the outer container can be discharged.

The container cover may be provided at one side thereof with a gas supply portion supplying a gas to the inside of the outer container. Further, the gas supply portion may be opened and closed as needed.

Further, the gas supply portion may additionally include a filter having a fine filtering structure.

The inner container includes a bottom surface to which cells can be adhered and a side surface of a mesh shape.

A specific aspect of the present invention is that the inner container includes a bottom surface and a side surface to which cells can be adhered, and the bottom surface and the side surface may be in a mesh shape.

The cell culture container is provided at the outside thereof with a liquid culture medium supply device and a gas supply device, and the liquid culture medium supply device is connected to the inlet portion and the gas supply device is connected to the gas supply portion.

Further, the outer container and the inner container are made of any one material selected from polystyrene (PS), polypropylene (PP) and acryl, and the outer container and the inner container may be formed of a material which is different from each other, respectively.

Further, the present invention relates to a circulation culture system, and the circulation culture system includes: a plurality of cell culture containers each having an outer container of which an upper surface is open and into which a culture medium can be held, a container cover coupled to an upper end of the outer container to prevent the penetration of microorganisms, and an inner container which is placed inside the outer container, can hold a culture medium, and can be separated from the outer container; circulation conduits connecting each of the adjacent cell culture containers so as to allow the plurality of the cell culture containers to be interconnected and connecting a pair of the cell culture containers placed at both ends; and a circulation section for supplying a culture medium and gas to a cell culture portion so as to circulate the same.

Further, the circulation section includes a pump connected to the circulation conduit to supply a fluid to the cell culture portion through the circulation conduit; a pump driving means for driving the pump; a weight sensor mounted to the culture container to detect varying weight values of the culture container during the culture process; and a flow control means for selectively operating any one of the pump driving means in response to a signal detected in the weight sensor.

At this time, the pump driving means may be further provided with a power supply portion to supply electric power to the pump driving means, and a receiving section for receiving the cell culture container.

Hereinafter, the present invention will be described in detail with reference to the accompanying drawings. While the invention will be described in conjunction with drawings, it should be understood that the present description is not intended to limit the scope of the present invention.

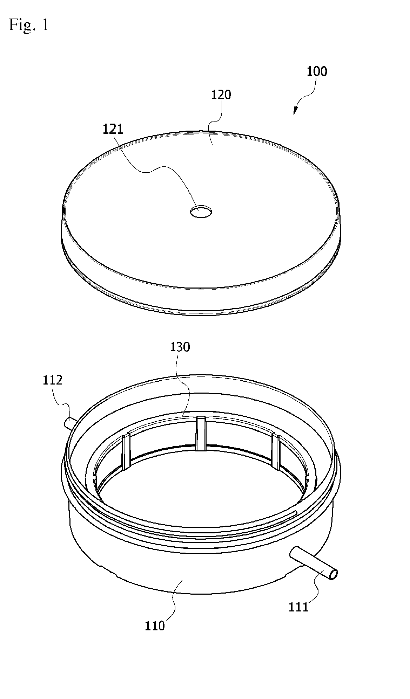

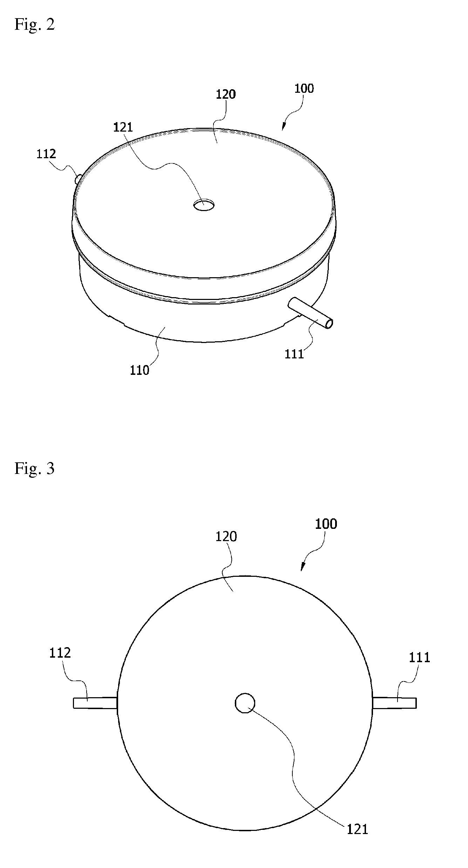

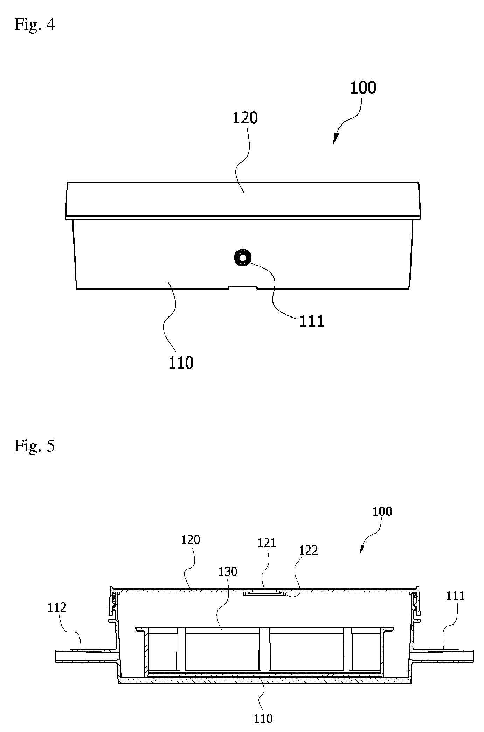

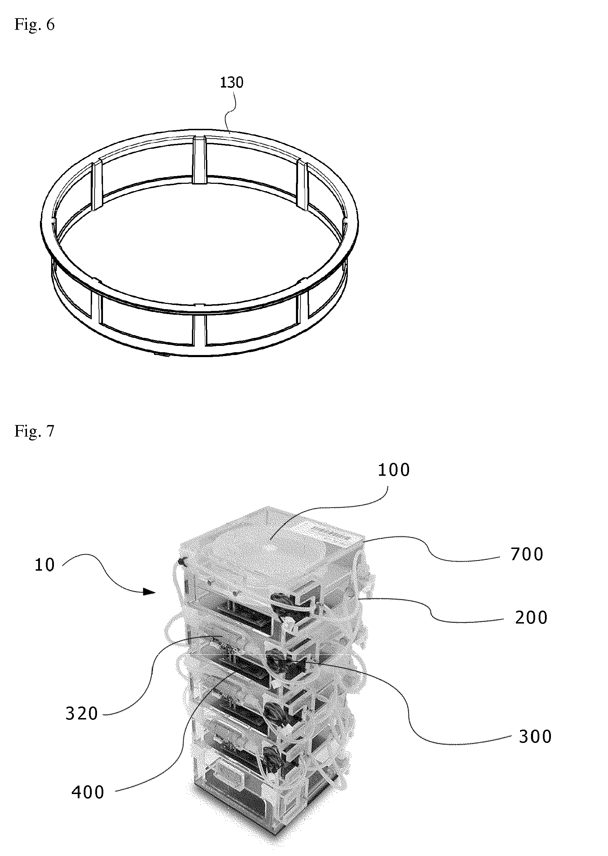

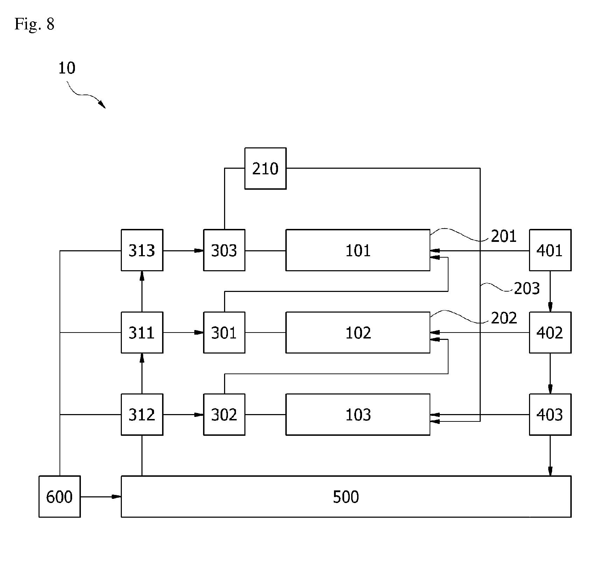

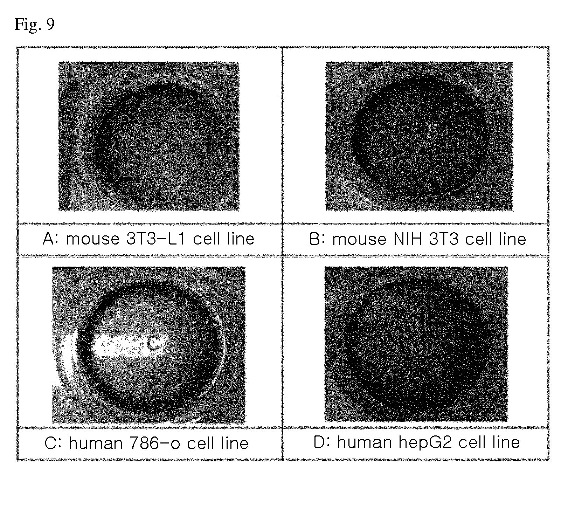

FIG. 1 is an exploded perspective view of a cell culture container according to the present invention. FIG. 2 is a perspective view of an outer container according to the present invention. FIG. 3 is a front view of the outer container according to the present invention. FIG. 4 is a right side view of the outer container according to the present invention. FIG. 5 is a sectional view of the cell culture container according to the present invention. FIG. 6 is a perspective view of an inner container according to the present invention. FIG. 7 is a configuration view of a circulation culture system according to the present invention. FIG. 8 is a configuration view of a circulation culture system according to an embodiment of the present invention. FIG. 9 is a view showing the results after various cells have been cultured using the circulation culture system according to an embodiment of the present invention.

The configuration and function of the cell culture container of the present invention will be described in detail with reference to FIGs. 1 to 9.

The present invention includes the inner container 130, which is comprised of a different material than that of the outer container and is placed inside the outer container 110 in which the culture medium can be held, and thereby it is possible to simultaneously culture the adherent cell and the non-adherent cell or two types of non-adherent cells. Further, the present invention is intended to provide the cell culture container 100 that makes it possible to observe the penetration and transfer of the cultured cells, of which the cell lines not having ordinary cells such as cancer cells that have penetration ability are cultured in the inner container 130, from the inner container 130 to the outer container 110, and that makes it convenient to inject and discharge the culture medium or gas from the outside thereof, thereby making it possible to culture the cells in any distinct environment.

In more detail, as shown in FIGs. 1 to 6, the cell culture container 100 according to the present invention includes the outer container 110, the container cover 120, and the inner container 130.

At this time, the outer container 110 is configured to have the upper surface that is open so that a culture medium can be held in the outer container 110, and the container cover 120 coupled to the upper end of the outer container 110 prevents the penetration of microorganisms. Further, the outer container 110 and the container cover 120 are transparent and made by injection-molding or blowing-molding a synthetic resin of elastic material.

Herein, the cell culture container 100 refers to a cell culture container 100 in which cells and tissues of humans or animals/plants or microorganisms can be cultured.

The outer container 110 according to the present invention is provided at one side thereof with the inlet portion 111, and at the other side thereof with the outlet portion 112. In more detail, the inlet portion 111 allows the culture medium to flow into the outer container 110, and the outlet portion 112 allows the culture medium which has flowed into the outer container 110 to be discharged outside of the outer container 110.

In particular, a cap (not shown) may be additionally provided for closing the inlet portion 111 and the outlet portion 112 while the cells are cultured.

Further, one or more inlet portion 111 and outlet portion 112 may be provided as is necessary.

The inlet portion 111 and the outlet portion 112 may be placed at opposite sides from each other so that a flow direction of the culture medium is distinguishable when the culture medium is injected or discharged.

At this time, the culture medium may be employed which is suitable for the object of use in the case where cell lines of animals (humans) or plants or microorganisms, which are not limited to any one of them, are cultured. For example, when the cell lines of animals (humans) are cultured, Dulbecco's Modified Eagle's Medium (DMEM) may be used.

Next, according to the present invention, the container cover 120 includes the gas supply portion 121.

In more detail, the gas supply portion 121 is a passage through which needed air can pass at the time of the culture, and may be provided inside thereof with a filter 122. The filter 122 serves to filter out microorganisms included in air passing through the passage so that the microorganisms cannot flow into the cell culture container 100 but rather only the air enters the culture container 100.

Herein, the gas supplied through the gas supply portion 121 may be oxygen or carbon dioxide. In more detail, in the case of animal cells, oxygen may be needed; in the case of plant cells, carbon dioxide may be needed. Of course, nitrogen or the other gases may be supplied as well.

A specific aspect of the present invention is that the gas supply portion 121 may be opened and closed. In more detail, in the case that the culture medium is supplied to the inlet portion 111 and discharged from the outlet portion 112, the gas is supplied through the gas supply portion 121; and in the case that the gas is supplied to the inlet portion 111 and discharged from the outlet portion 112, the gas supply portion 121 is blocked and the cells can be cultured. For example, a common adhesive tape is used to block the gas supply portion 121.

For example, in the case that cells, plants or microorganisms are cultured under a specific environment such as a hypoxia environment, the gas supply portion 121 provided in the container cover 120 is blocked and the mixed gas of hypoxia may be discharged through the culture medium inlet portion 111 and outlet portion 112 of the outer container 110. At this time, the inner container 130 is put in the general cell culture container 100 and filled with the culture medium, and then they can be held in the outer container 110 provided in the present invention, thereby forming a culture container having a three-dimensional structure.

Further, a culture medium supply device and a gas supply device may be connected to the outer side of the cell culture container 100, thereby supplying the culture medium and gas to the inside of the culture container. At this time, the gas supply device may be a common gas control device, e.g., the Herrison gas controller.

As shown in FIG. 6, the inner container 130 according to the present invention may include a bottom surface and a mesh shaped side surface to which cells can be adhered. Since the side surface of the inner container 130 is mesh-shaped, the cell culture can be performed even on the side surface, thereby facilitating the three-dimensional cell culture. Particularly, since the present invention facilitates the three-dimensional cell culture, the present invention serves to provide an in vivo mimic environment and reduce any difference between in vitro culture and in vivo culture. Furthermore, the present invention may serve to reduce the number of test animal used.

In addition thereto, the inner container 130 is placed inside the outer container 110, thereby contributing to prevent two-different types of cells from being mixed during culturing.

Further, although mesh shape refers to a mesh-like shape, the shape is not limited thereto if the shape corresponds to a shape capable of preventing the cells cultured in the outer container 110 and the inner container 130 from being mixed.

A specific aspect of the present invention is that the inner container 130 includes a bottom surface and a side surface that enable cells to adhere thereto, and the bottom surface and the side surface may be mesh-shaped. At this time, the outer side of the bottom surface of the inner container 130 is coated with fibronectin and the cells mixed with methylcellulose can be cultured on the inside of the bottom surface. Such a structure may be effective in terms of observing the penetration and transfer of the cells. Further, it enables observation of whether there is any effect caused by signal transduction factors output to the outside, and observation of the penetration and transfer of the cells by means of a microscope without any other operations.

Herein, the fibronectin is a glycoprotein that exists on the surfaces of normal fibroblast or endotheliocyte, the basement membrane of intestinal epithelial cells, and the adhesive surfaces of other various cells, and refers to the components of perithelium. Fibronectin has various functions, but in the present invention it helps promote the adhesive property and stretching property of the culture cells on the Petri dish.

Further, the mesh-typed inner container 130 enables the separation of a colony forming cell that bunches together to grow and a single cell that does not, and enables the single cell to be easily separated from the tissue.

Further, the outer container 110 and the inner container 130 according to the present invention may be made of any one selected from polystyrene (PS), polypropylene (PP) and acryl.

In particular, the outer container 110 and the inner container 130 may be made of material which is different from each other, respectively. Thus, an adherent cell and a non-adherent cell or two different types of non-adherent cells can be simultaneously cultured.

According to an embodiment of the present invention, the outer container 110 is made of polypropylene (PP) and the inner container 130 is made of polystyrene (PS). At this time, the outer container 110 serves to culture the non-adherent cells and the inner container 130 serves to culture the adherent cells. Further, cell signal transduction factors are secreted to the culture medium, which makes it possible to observe the interaction thereof.

Herein, the cell signal transduction factor may refer to a growth factor, cytokine or a vascular endothelial growth factor (VEGF). If a factor corresponds to the cell signal transduction factor satisfying the object of the present invention, the factor is naturally included in the scope of the present invention.

Further, in the case that the outer container 110 is made of polypropylene (PP), the container is stronger than a conventional cell culture dish, and thus can be reused after being washed and sterilized under high pressure. Further, since it can be reused, it is possible to culture the non-adherent cell more easily than the adherent cell as well as reduce cost. However, in the case that polypropylene (PP) is used, since the adherence of the adherent cell to the container becomes possible by a simple coating of polyglycine thereon, it is possible to simultaneously culture adherent cells which are different from each other in the outer container 110 and the inner container 130 respectively, and thus it is also possible to simultaneously culture two different types of non-adherent cells.

Further, the inner container 130 according to the present invention can be easily separated from the outer container 110. Therefore, as a result of the separation of the inner container 130 from the outer container 110, it is possible to conveniently transfer the container to other culture conditions, and it is easy to collect the cells of the inner container 130 and the outer container 110 separately.

In addition, the dual structure culture container 100 according to the present invention may be configured to have a diameter of 5 to 20 cm and a height of 1 to 3 cm, but is not limited as a culture container for cell culture. For example, the present invention may employ a dual structure culture container having a diameter of 5 to 6cm and a height of 1cm, or a dual structure culture container having a diameter of 9 to 10cm and a height of 1.5 cm. Further, the receiving portion 700 receives the dual structure culture container 100 and the size is determined depending on the size of the dual structure culture container 100, but is not limited to any specific size as long as the receiving portion is capable of receiving the dual structure culture container 100.

In addition, the present invention relates to a circulation culture system.

In particular, the circulation culture system according to the present invention includes a cell culture container 100, a circulation conduit 200 and a circulation section.

The cell culture container 100 may be a dual structure cell culture container 100 including the outer container 110 and the inner container according to the present invention. Particularly, the cell culture container 100 may be formed with a plurality of containers 100, and the circulation conduit 200 serves to connect each of the adjacent cell culture containers so that the plurality of the cell culture containers 100 is interconnected and to connect a pair of the cell culture containers placed at both ends thereof

Herein, the cell culture containers placed at both ends refer to a pair of the cell culture containers 100 placed at the outermost sides of the plurality of the cell culture containers 100.

As shown in FIGS. 7 and 8, the cell culture portion according to the present invention may include, for example, three containers referred to as a first cell culture container 101, a second cell culture container 102 and a third cell culture container 103.

At this time, the first cell culture container 101 is connected to the second cell culture container 102 by means of the circulation conduit 200. For convenience, the circulation conduit 200 connecting the first cell culture container 101 and the second cell culture container 102 is referred to as a first circulation conduit 201, the circulation conduit 200 connecting the second cell culture container 102 and the third cell culture container 103 is referred to as a second circulation conduit 202, and the circulation conduit 200 connecting the third cell culture container 103 and the first cell culture container 101 is referred to as a third circulation conduit 203.

Particularly, the circulation conduit 200 may be connected at one side thereof to the inlet portion 111 of the cell culture container, and at the other side thereof to the outlet portion of the adjacent cell culture container. As an example, the first circulation conduit 201 may be connected to the inlet portion 111 of the first culture portion and the outlet portion of the second cell culture container 102.

Further, the circulation conduit 200 may include a flow sensor 320 additionally. The flow sensor 320 serves to maintain a constant flow-rate and flow-velocity of the culture medium when the culture medium is circulated in the cell culture container 100. As an example, the flow sensor 320 may be a pressure resistant milliliter flow sensor.

Further, the circulation conduit 200, which connects the pair of the cell culture containers placed at both ends, may include at least one fluid shut-off valve 210. For example, it may be included in the third circulation conduit 203.

Herein, the fluid shut-off valve 210 shuts off the circulation of fluid, and may be a conventional stop valve.

In addition, the circulation culture system according to the present invention may include a circulation section capable of supplying a culture medium and a gas to the cell culture container.

In particular, the circulation section, which is provided to continuously circulate the culture medium in the plurality of the cell culture portions, may include a pump 200, a pump driving means 310, a weight sensor 400 to detect varying weight values in the culture container, and a fluid control means 500 to selectively operate any one of the pump driving means in response to signals detected in the weight sensor 400.

Herein, the pump 200 is provided to circulate the culture medium in the cell culture container, which may be provided with at least one or a plurality of pumps 200 so as to correspond to a plurality of cell culture containers.

In addition, the pump 200 and the pump driving means 310 for driving the pump 200 may be provided between the plurality of the culture containers so as to supply fluid to the culture containers through the circulation conduit 200, and may be connected to the circulation conduit 200. For example, the first circulation conduit 201 may be connected to the first pump 301, the second circulation conduit 202 may include the second pump 302 and the third circulation conduit 203 may include the third pump 303. In addition thereto, for example, the first pump driving means 311 for driving the first pump 301, the second pump driving means 312 for driving the second pump 302 and the third pump driving means for driving the third pump 303 may be added thereto.

As another example, the pump 200 employed in the present invention may be a piezo pump. The piezo pump serves to convert an electric energy to a mechanical energy using the piezoelectric effect which circulates fluid. That is, the piezo pump can apply pressure to fluid using the piezoelectric transducer.

When the piezoelectric transducer is applied with a voltage, the electrostrictive effect by which the piezoelectric crystal forming the piezoelectric transducer is distorted is generated. The piezo pump serves to transfer an air gas as a fluid in a predetermined direction by using the mechanical energy generated due to displacement of the piezoelectric crystal as a result of the electrostrictive effect.

In the present invention, the piezoelectric transducer refers to the pump driving means 310.

Particularly, the pump 200 is driven by the pump driving means 310. The pump driving means 310 may be operated in response to a signal output from the weight sensor 400 mounted to the culture container, thereby driving the pump 200.

The weight sensor 400, which is mounted to the culture container, is a sensor for detecting a weight of fluid in the culture container to convert the detected weight to an electric signal and transmit the signal to a signal processor of the fluid control means 500. At this time, the weight sensor 400 mounted to the first culture container is referred to as a first weight sensor 401, the weight sensor 400 mounted to the second culture container is referred to as a second weight sensor 402, and the weight sensor 400 mounted to the third culture container is referred to as a third weight sensor 403. In the present invention, the weight scope detected by the weight sensor 400 may be in the range of 0.2 to 130 g or 10 to 80g.

Particularly, the present invention includes the fluid control means 500, and the fluid control means 500 serves to selectively operate any one of the plurality of the pump driving means 310 in response to a signal detected in the weight sensor 400.

The culture medium is sufficiently mixed while being circulated sequentially or selectively along the circulation conduit 200 and the cell culture portion, thereby enabling a high level of productivity.

The circulation culture system serves to circulate the culture medium through the circulation section, and at the same time, is provided in the cell culture portion with a temperature sensor, thereby enabling setting and maintaining a temperature suitable for culturing cells or tissues.

For example, the temperature may be set in the range of 30 to 50°C or 36 to 44°C thereby enabling the maintaining of the temperature of the culture medium.

In addition thereto, the circulation culture system according to the present invention further includes a receiving portion capable of receiving the cell culture portion. As shown in FIG. 7, the receiving portion is configured to enable the plurality of the cell culture portions to be easily stacked.

At this time, the receiving portion may be formed in a shelf shape so as to easily receive the plurality of cell culture containers 100, and may be provided with holes at positions corresponding to the inlet portion 111 and outlet portion of the cell culture container 100. The plurality of cell culture portions can be easily connected to the circulation conduit 200 through the holes.

Further, at least one of the plurality of cell culture containers 100 may be provided with a fluid supply portion capable of supplying a fluid into the culture container, and may additionally be provided with a pump 300, a pump driving means 310, a weight sensor 400 and a power supply portion 600 to supply power to the flow control means 500.

In addition thereto, the flow control means 500 includes a time control setting portion to set time control through the time control setting portion, an operation state portion to generate a stop alarm when the operation of the circulation culture system is completed or generate a failure alarm when the pump 300 or the weight sensor 400 fails to operate.

Hereinafter, the effects of the present invention will be described in detail with reference to test example.

### <Example>

### Example 1.

Cell culture of various types using a circulation culture system according to the present invention.

Four types of cells are cultured in order to observe the three-dimensional culture according to an embodiment of the present invention.

First, four types of cells such as NIH 3T3 cell line (ATCC) and 3T3-L1 (ATCC) preadipocytes, as normal fibrous cells of a mouse, and 786-o (ATCC) renal cancer cells, and hepG2 (ATCC) hepatocarcinoma cells, as human cells, are cultured two-dimensionally in the culture dish of the present invention.

Next, these cells are treated with trypsin (Gibco, USA) so that they are separated into single cells, and then mixed with methylcellulose (methylcellulosl.4%, R&D, USA) to satisfy 5×10⁴cell/mL, and thereafter, the four types of cells are each planted in the dual structure culture container according to the present invention for the culture preparation. In particular, the cells are planted in each of the outer container 110 and the inner container 130 of the dual structure culture containers.

Thereafter, each of the four dual structure culture containers is placed in the receiving portions of the circulation culture system, and then, the cells are cultured at 37°C for 72 hours while the culture medium is circulated at the flow speed of 7mL/min. Thereafter, the cultured cells are dyed with haematoxylin (Sigma, USA) so that the survival and cultured state of the cells can be examined.

As a result, as shown in FIG. 6, it can be seen that the cells were cultured three-dimensionally on the side surface of the inner container 130.

Further, by using the cell culture system according to the present invention, the three-dimensional culture can be easily performed as a result of sequentially, selectively, or quantitatively controlling and circulating the culture medium.

As in the foregoing, the present invention has been described in detail with reference to the preferred embodiments thereof. However, it will be appreciated by those skilled in the art that various changes and modifications may be made in these embodiments without departing from the principles and spirit of the invention. Accordingly, the scope of the present invention is defined in the appended claims and their equivalents.

### <Description of reference numerals>

100: cell culture container, 101: first cell culture container
102: second cell culture container, 103: third cell culture container
110: outer container
111: inlet portion, 112: outlet portion
120: container cover
121: gas supply portion, 122: filter
130: inner container
200: circulation conduit, 201: first circulation conduit
202: second circulation conduit, 203: third circulation conduit
210: fluid shut-off valve
300: pump, 301: first pump
302: second pump, 303: third pump
310: pump driving means, 311: first pump driving means
312: second pump driving means, 313: third pump driving means
320: flow sensor
400: weight sensor, 401: first weight sensor
402: second weight sensor, 403: third weight sensor
500: flow control means
600: power supply portion
700: receiving portion

## Claims

1. A cell culture container, comprising:
an outer container of which an upper surface is open and into which a culture medium can be held;
a container cover coupled to an upper end of the outer container to prevent penetration of microorganisms; and
an inner container which is placed inside the outer container, can hold a culture medium, and can be separated from the outer container.

2. The cell culture container of claim 1, wherein the outer container is provided at one side surface thereof with an inlet portion through which a culture medium or gas can flow into the outer container; and at the other side surface thereof with an outlet portion through which the culture medium or gas which has flowed into the outer container can be discharged.

3. The cell culture container of claim 1, wherein the container cover is provided at one side thereof with a gas supply portion supplying gas inside the outer container.

4. The cell culture container of claim 3, wherein the gas supply portion can be opened and closed.

5. The cell culture container of claim 3, wherein the gas supply portion further includes a filter having a fine filtering structure.

6. The cell culture container of claim 1, wherein the inner container includes a bottom surface to which cells can be adhered and a side surface of a mesh shape.

7. The cell culture container of claim 1, wherein the inner container includes a bottom surface and a side surface to which cells can be adhered, and the bottom surface and the side surface are formed in a mesh shape.

8. The cell culture container of claim 1, wherein the cell culture container is provided at an outside thereof with a culture medium supply device and a gas supply device.

9. A circulation culture system, comprising:
a cell culture container having an outer container of which an upper surface is open and into which a culture medium can be held, a container cover coupled to an upper end of the outer container to prevent the penetration of microorganisms, and an inner container which is placed inside the outer container, can hold a culture medium, and can be separated from the outer container; and having a plurality of cell culture containers;
circulation conduits that connect each of the adjacent cell culture containers so as to allow the plurality of the cell culture containers to be interconnected and that connect a pair of the cell culture containers placed at both ends; and
a circulation section that supplies a culture medium and gas to the cell culture portion so as to circulate the same.

10. The circulation culture system of claim 9, wherein the circulation section comprises a pump connected to the circulation conduit to supply a fluid to the cell culture portion through the circulation conduit;
a pump driving means for driving the pump;
a weight sensor mounted to the culture container to detect varying weight values of the culture container during a culture process; and
a flow control means for selectively operating any one of the pump driving means in response to the signal detected in the weight sensor.

11. The circulation culture system of claim 9, wherein the pump driving means is provided with a power supply portion for supplying electric power to the pump driving means.

12. The circulation culture system of claim 9, further comprising a receiving portion for receiving the cell culture container.
